# EUROPEAN PATENT SPECIFICATION

(11) **EP 0 577 009 B1**
(45) Date of publication and mention of the grant of the patent: **29.09.1999**
(21) Application number: 93110096.0
(22) Date of filing: 24.06.1993
(51) Int. Cl.: A61L 29/00, A61L 31/00, A61L 27/00

(54) **Shaped articles for biomedical use**
Formkörper zur biomedizinische Verwendung
Articles façonnés à usage biomédical

(30) Priority: 30.06.1992 IT MI921593
(43) Date of publication of application: 05.01.1994
(73) Proprietor: Montell Technology Company bv, 2132 MS Hoofddorp (NL)
(72) Inventor: Galimberti, Maurizio, I-20137 Milano (IT); Martini, Emilio, I-40037 Sasso Marconi (BO) (IT); Albizzati, Enrico, I-28041 Arona (NO) (IT)
(74) Representative: Zanoli, Enrico

(56) References cited:
- FR-A- 2 537 981
- GB-A- 2 053 246

## Description

The present invention relates to shaped articles for biomedical use prepared starting from a polymeric material.

The material most used for the manufacturing of flexible devices for biomedical use is represented by plastified PVC. High flexibility characteristics of the plastified PVC are due to plasticizers contained in it.

The migration and extractability of plasticizers in biologic liquids, with consequent negative effects on the health of the patient because of the toxicity of plasticizers, represent an essential problem for PVC that until now has not yet been solved. Furthermore, the extraction of plasticizers worsens the mechanical properties of PVC and diminishes its flexibility.

In addition, it has to be kept in mind the problem of the surface migration of additives based on organopolysiloxane oils used in PVC as external lubricant to give to the PVC surface a critical surface tension in order to avoid blood coagulation phenomena. Organosiloxane compounds in fact have the tendency to bleed from the PVC surface thus giving rise to problems deriving from their poor compatibility with blood (see European Patent Application EP-A-0287482).

Therefore, the need for materials which may replace PVC in these fields is felt.

Other materials useable for manufacturing flexible devices for biomedical use are styrene-ethylene-butene-styrene (SEBS) block copolymers. SEBS modified by polysiloxanes have been suggested for example as substitutive of PVC and silicone resins for the realization of some devices, such as endotracheal tubes (US Patent 4,386,179).

These copolymers combine good properties of optical transparency and flexibility also at low temperatures.

US Patent 4,335,225 describes the preparation of high molecular weight polypropylene having elastomeric characteristics and processable by technologies for thermoplastic materials and proposes a possible use for this material in the manufacture of some articles for biomedical use. The polymerization is carried out by using as catalyst the reaction product of an organic derivative of zirconium, generally tetraneophilzirconium, with a hydroxylated alumina. However, the aluminium content in the polymer which is obtained is very high, higher than 1000 ppm. The above mentioned materials till now have not found any significant application in the biomedical field.

Now it has been unexpectedly found a polymeric material suitable for the manufacture of articles for biomedical applications which combines a good set of mechanical properties with good optical properties, and that furthermore does not present problems deriving from the exctractability of metals in biologic fluids.

The material consists of elastomeric copolymers of ethylene with propylene and/or other alpha-olefins or polyenes having an ethylene content comprised between 45 and 85% by mols, preferably between 50 and 75% by mols, propylene and/or alpha-olefins CH₂=CHR, wherein R is an alkyl radical having 2-10 carbon atoms, content between 15 and 55% by mols, preferably between 25 and 50% by mols, and diene or polyene content between 0 and 10% by mols, characterized by the following properties:
- solubility in pentane at 25°C higher than 90%;
- substantial absence of crystallinity (melting enthalpy lower than 20 J/g);
- content of propylene units or units deriving from the alpha-olefin in form of triads comprised between 4 and 50% of propylene or alpha-olefin and at least 70% of said triads showing isotactic structure;
- random distribution of ethylene and propylene units and/or units deriving from alpha-olefins in segments of the chain having copolymeric structure (values of the product of reactivity ratios of ethylene and propylene or alpha-olefin comonomers comprised between 0.4 and 1).

Copolymers have inherent viscosity higher than 1.5 dl/g, preferably higher than 2 dl/g and generally comprised between 2.5 and 3.5 dl/g. Particularly preferred are copolymers having an intrinsic viscosity between 2.5 and 3.0 dl/g.

The content of diene or polyene units is preferably comprised between 0.5 and 5% by mols.

Copolymers can be transformed into manufactured articles by usual manufacturing processes of thermoplastic materials (moulding, extrusion, injection, e.g.) and relative manufactured articles are endowed with elasto-plastic properties particularly interesting for biomedical articles.

High elasto-plastic properties of copolymers are made clear by low tension set values at 200%, 1 min., 25°C (values are lower than 30%, and generally comprised between 10 and 20%) and by a ultimate tensile strength higher than 4 MPa and generally comprised between 5 and 10 MPa. The mentioned values relate to tests carried out according to ASTM D412.

Optical properties are evaluated by measuring , on a plate having 1 mm thickness, the quantity of transmitted light which deviates from the original incidence angle ("haze"). Copolymers used for the preparation of the shaped articles of the invention are characterized by haze values generally lower than 40%, preferably lower than 25%.

Copolymers are prepared by polymerization of ethylene with propylene and/or an alpha-olefin CH₂=CHR, wherein R is an alkyl having from 2 to 10 carbon atoms, optionally in the presence of a diene or polyene, with chiral catalysts obtained from metallocene derivatives of zirconium, such as ethylene-bis-(tetrahydroindenyl)zirconium dichloride or dimethylsilanylen-bis-(tetrahydroindenyl)-zirconium dichloride and from tetraisobutylalumoxane, according to what is described in International document WO 93/19107 published on 30 September 1993.

The copolymerization is carried out in liquid phase consisting of propylene and/or alpha-olefin, working at a temperature of 40 to 50°C.

Usable alpha-olefins are, for example, butene-1, pentene-1, 4-methyl-pentene-1, hexene-1, octene-1, dodecene-1. Dienes or polyenes usable are preferably selected among linear non conjugated diolefins, such as 1,4-hexadiene, or cyclic having an internal bridge, such as 5-ethylidene-2-norbornene.

Copolymers obtained are characterized by a very low content of metals; generally the quantity of zirconium is lower than 1 ppm and the quantity of aluminium is lower than 500 ppm and it is generally comprised between 40 and 100 ppm. However, it is possible to obtain copolymers having even lower residue quantities of metals. Furthermore, metals contained are practically not extractable by contact with biological fluids, which is particularly advantageous in biomedical applications.

These copolymers show a unique combination of properties, in particular flexibility at low temperatures (-40°C and lower), thermoplastic processing and optical transparency. Furthermore, these copolymers have a good compatibility with blood and soft tissues.

In view of these properties, copolymers are particularly suitable for the manufacture of articles for biomedical applications.

For biomedical applications articles are meant those articles intended for the contact with biologic fluid or injectable. Examples of manufactured articles according to this invention are tubes for enteral and parenteral feeding, tubes for peristaltic pumps, catheters, devices for hemodialysis, bags for blood or plasma, syringe seals, artificial organs and similar applications.

Owing to the transparency characteristics of copolymers used for the preparation of the articles object of the invention,( haze values even lower than 20%), devices for containing, administering, draining and conveying of blood and physiological fluids, such as intravenous catheters, tubes for dialysis, bags for blood and physiological solutions and similar applications are particularly interesting. In fact it is easy to detect inside the device the presence of bubbles, blood clots, scales of biomineral source in dialysis devices or the presence of extraneous material.

The sterilization by radiation treatment, in particular gamma radiation, according to known technologies or chemically (aseptic Sterilization), the resistance to solvents used in hospitals, non adsorbance of drugs, weldability according to known welding techniques, the dimentional stability are further interesting characteristics of the manufactured articles of the invention.

Further advantages are clear from the examples which are given in order to describe and not to limit the invention.

### Examples 1 and 2

Ethylene-propylene copolymers containing 69% (Example 1) and 75% (Example 2) by mols respectively of ethylene have been prepared according to the process described in WO-A-9319107, using a catalytic component obtained from ethylene-bis-(tetrahydroindenyl)-zirconium dichloride and tetraisobutyl-alumoxane. Copolymers obtained show intrinsic viscosities between 2.5 and 3.0 dl/g.

The characterization of copolymers have been carried out on specimens prepared by pressure die-casting, using a cooling speed from 200°C to room temperature of 40°C/min in order to approach the cooling generally used in producing manufactured articles. Experimental data are reported in Table 1.

**TABLE 1**

| Example | 1 | 2 |
|---|---|---|
| Ethylene content (% by mol) | 69 | 75 |
| Ultimate tensile strength^{(a)} (MPa) | 6.7 | 8.5 |
| Ultimate elongation^{(a)} (%) | 650 | 440 |
| Torsional elastic modulus^{(b)} (MPa) | 6.3 | 11.2 |
| Haze (c) (%) | 17 | 29 |
| Shore A (d) | 41 | 46 |
| Tension set^{(a)} (%) | 12 | 16 |

| | | |
|---|---|---|
| ^{(a)}ASTM D412 | | |
| ^{(b)}ASTM D4065 | | |
| ^{(c)} Thickness 1 mm | | |
| ^{(d)} Reading after 5 seconds | | |

Measure of the torsional elastic modulus has been carried out at a frequency of 10 Hz with a DMTA of Polymer Lab. on specimens 10 mm width, thickness 1.5 mm and length of the useful segment 20 mm.

The suitability of copolymers of examples 1 and 2 for the production of articles for biomedical applications has been experimentally proved by overcoming the following tests, carried out on tubular specimens having inside diameter ID=2.6 mm, outside diameter OD=3.6 mm and thickness t=0.5 mm.
- Stretching test:: A specimen of length L=61 cm is stretched by hand until its length is doubled. The overcoming of the test is indicated by the absence of "cracks".
- Knot Test:: A simple knot is produced from a specimen having length about 30.5 cm, free ends are slowly stretched by tighting the knot. At the end of overcoming this test, walls of the tube must not stick to each other in such a way as to block the flow of liquids through the tube itself.
- Kink Test:: The tube is maintained bent by means of a V type clamp for 6 hours at room temperature and therafter it is freed. The tube must not remain obstructed and must not show bends, nor necks.

As already said, metals contained in copolymers used in the invention are essentially not extractable by contact with biological fluids, which is particularly advantageous in biomedical applications.

## Claims

1. Shaped articles for biomedical use prepared starting from elastomeric copolymers of ethylene with propylene and/or alpha-olefins CH₂=CHR, wherein R is an alkyl containing from 2 to 10 carbon atoms, and optionally with lower proportions of units deriving from a diene or polyene, containing from 45 to 85% by mols of ethylene, from 15 to 55% by mols of propylene and/or alpha-olefins, and from 0 to 10% by mols of diene or polyene, characterized by the following properties:
- crystallinity content, measured as melting enthalpy, lower than 20 J/g;
- solubility in pentane at 25°C higher than 90%;
- content of propylene units or units deriving from alpha-olefin in form of triads comprised between 4 and 50% of propylene or alpha-olefin and percentage of said triads having isotactic structure of at least 70%;
- product r₁.r₂ of the reactivity ratio of ethylene, r₁, and of propylene or alpha-olefin, r₂, comprised between 0.4 and 1;
- intrinsic viscosity higher than 1.5 dl/g.

2. Shaped articles according to claim 1 wherein the copolymers are characterized by an intrinsic viscosity comprised between 2.5 and 3.0 dl/g.

3. Shaped articles according to claim 1 wherein the copolymers contain from 50 to 75% by mols of ethylene.

4. Shaped articles according to claim 1 wherein the copolymers are characterized by a tension set lower than 30% and an ultimate tensile strength higher than 4 MPa.

5. Shaped articles according to claim 1 wherein the copolymers are characterized by haze values lower than 40%.

6. Shaped articles according to claim 1 characterized by the fact that they are selected among: devices for containing, administering, draining or conveying blood and biological or physiological fluids; tubes for enteral or parenteral feeding; catheters; syringe seals and artificial organs.

## Patentansprüche

1. Formgegenstände zur biomedizinischen Verwendung, hergestellt ausgehend von elastischen Copolymeren von Ethylen mit Propylen und/oder α-Olefinen CH₂=CHR, worin R einen Alkylrest darstellt, der 2 bis 10 Kohlenstoffatome enthält, und gegebenenfalls mit geringeren Anteilen an Einheiten, die von einem Dien oder Polyen abgeleitet sind, die 45 bis 85 Mol-% Ethylen, 15 bis 55 Mol-% Propylen und/oder α-Olefine und 0 bis 10 Mol-% Dien oder Polyen enthalten, gekennzeichnet durch die nachstehenden Eigenschaften:
- Kristallinitätsanteil, gemessen als Schmelzenthalpie, geringer als 20 J/g;
- Löslichkeit in Pentan bei 25°C höher als 90%;
- Anteil an Propyleneinheiten oder Einheiten, abgeleitet von α-Olefin, in Form von Triaden, umfaßt zwischen 4 und 50% Propylen oder α-Olefin, und Prozentsatz der Triaden mit isotaktischer Struktur von mindestens 70%;
- Produkt r₁.r₂ des Reaktivitätsverhältnisses von Ethylen, r₁, und Propylen oder α-Olefin, r₂, umfaßt zwischen 0,4 und 1;
- Grenzviskosität höher als 1,5 dl/g.

2. Formgegenstände nach Anspruch 1, wobei die Copolymere durch eine Grenzviskosität, umfaßt zwischen 2,5 und 3,0 dl/g, gekennzeichnet sind.

3. Formgegenstände nach Anspruch 1, wobei die Copolymere 50 bis 75 Mol-% Ethylen enthalten.

4. Formgegenstände nach Anspruch 1, wobei die Copolymere durch einen Zugverformungsrest unterhalb 30% und eine Reißfestigkeit höher als 4 MPa gekennzeichnet sind.

5. Formgegenstände nach Anspruch 1, wobei die Copolymere durch Haze-Werte unterhalb 40% gekennzeichnet sind.

6. Formgegenstände nach Anspruch 1, dadurch gekennzeichnet, daß sie ausgewählt sind aus: Vorrichtungen zum Enthalten, Verabreichen, Ableiten oder Weiterleiten von Blut und biologischen oder physiologischen Flüssigkeiten, Röhren zur enteralen oder parenteralen Zuführung, Kathetern, Spritzenverschlüssen und künstlichen Organen.

## Revendications

1. Articles manufacturés pour l'usage biomédical, obtenus à partir de copolymères élastomères de l'éthylène avec le propylène et/ou des alpha-oléfines CH₂=CHR, où R est un alkyle contenant de 2 à 10 atomes de carbone, et le cas échéant, avec des proportions moindres de motifs dérivant d'un diène ou d'un polyène, contenant de 45 à 85 % molaires d'éthylène, de 15 à 55 % molaire de propylène et/ou d'alpha-oléfines et de 0 à 10 % molaires de diène ou de polyène, caractérisé par les propriétés suivantes :
- teneur en cristallinité, mesurée par l'enthalpie de fusion, inférieure à 20 J/g ;
- solubilité dans le pentane à 25°C supérieure à 90 % ;
- teneur en motifs propylène ou motifs dérivant de l'alpha-oléfine sous forme de triades comprise entre 4 et 50 % de propylène ou d'alpha-oléfine et pourcentage en lesdites triades ayant une structure isotactique d'au moins 70 % ;
- le produit r₁.r₂ du rapport de réactivité de l'éthylène, r₁, et du propylène ou de l'alpha-oléfine, r₂, compris entre 0,4 et 1 ;
- viscosité intrinsèque supérieure à 1,5 dl/g.

2. Articles manufacturés selon la revendication 1, dans lesquels les copolymères sont caractérisés par une viscosité intrinsèque comprise entre 2,5 et 3,0 dl/g.

3. Articles manufacturés selon la revendication 1, dans lesquels les copolymères contiennent de 50 à 75 % molaires d'éthylène.

4. Articles manufacturés selon la revendication 1, dans lesquels les copolymères se caractérisent par une déformation permanente en traction inférieure à 30 % et une résistance à la traction supérieure à 4 MPa.

5. Articles manufacturés selon la revendication 1, dans lesquels les copolymères se caractérisent par des valeurs de voile inférieures à 40 %.

6. Articles manufacturés selon la revendication 1, caractérisés par le fait qu'ils sont choisis parmi : dispositifs pour renfermer, administrer, prélever ou transporter du sang et des fluides biologiques ou physiologiques ; tubes pour administration entérale ou parentérale ; cathéters ; joints de seringues et organes artificiels.
